# EUROPEAN PATENT SPECIFICATION

(11) **EP 2 459 153 B1**
(45) Date of publication and mention of the grant of the patent: **11.09.2013**
(21) Application number: 10752402.7
(22) Date of filing: 29.07.2010
(51) Int. Cl.: A61Q 5/02, A61Q 5/00, A61K 8/02, A61K 8/11, A61K 8/41, A61Q 5/12

(54) **PHARMACEUTICAL OR COSMETIC OR DIETETIC COMPOSITION FOR PROMOTING A HAIR PIGMENTATION EFFECT**
PHARMAZEUTISCHE, KOSMETISCHE ODER ERNÄHRUNGSZUSAMMENSETZUNG ZUR FÖRDERUNG EINES HAARPIGMENTIERUNGSEFFEKTS
COMPOSITION PHARMACEUTIQUE OU COSMÉTIQUE OU DIÉTÉTIQUE AYANT POUR EFFET DE FAVORISER LA PIGMENTATION CAPILLAIRE

(30) Priority: 29.07.2009 IT MI20091361
(43) Date of publication of application: 06.06.2012
(73) Proprietor: Giuliani S.p.A., 20129 Milano (IT)
(72) Inventor: PAUS, Ralf, 22339 Hamburg (DE); GIULIANI, Giammaria, I-20123 Milano (IT); RAMOT, Yuval, 90805 Mevasseret-Zion (IL); BECKER, Astrid, 23564 Lübeck (DE); BARONI, Sergio, I-24030 Villa D'adda (IT)
(74) Representative: Appoloni, Romano
(86) International application number: PCT/IB2010/053450
(87) International publication number: WO 2011/013087

(56) References cited:
- WO-A1-03/063851
- WO-A1-2005/013932
- WO-A1-2006/048671
- WO-A2-2008/114141
- JP-A- 2008 156 330
- US-A1- 2009 070 945

## Description

### FIELD OF THE INVENTION

The present invention relates to hair pigmentation in man.

### BACKGROUND ART

The human hair follicle is a complex organ wherein there are interactions of epithelial (for example, different lines of keratinocytes, endothelium), mesenchymal (for example dermal papilla fibroblasts, connective tissue sheath fibroblasts), neuroectodermal (nerves, melanocytes) cellular populations and transitory migrating cells (immune cells, mastocytes).

The growth and pigmentation of hair fibers are affected by several intrinsic factors comprising changes depending on hair cycle, body distribution, age and genre differences, variable hormone sensitivity, genetic defects and age-related changes. The study of hair growth is also complicated by the effects of extrinsic variables that comprise climate and seasons, polluting substances, toxins and exposure to chemicals. The differences found between the pigmentation regulation in the epidermis and in hair follicles reflect the division into compartments of the mammal skin pigmentation system.

The melanocytes of epidermis, of the hair follicle bulb and of the sheath of the hair follicle outer root are very different from each other, despite the fact that the skin pigmentation in mammals must be understood as an open system. The major differences are those of the nature of their respective melanocyte-keratinocyte functional units. The melamine unit of the hair bulb is found in the proximal anagen bulb, which is an immunologically distinct region of skin and overall, is formed by one melanocyte every 5 keratinocytes in the hair bulb and of one melanocyte for each keratinocyte in the basal layer of the hair bulb matrix. On the contrary, each epidermal melanocyte is associated to 36 vital keratinocytes in the immunocompetent epidermal melamine unit.

However, the most obvious difference between these two melanocyte skin populations and with considerable implications for the regulation of the hair pigmentation, is the observation that the hair bulb melanocyte activity is subject to a cyclic control and that melanogenesis is strictly associated to the hair growth cycle. On the other hand, the skin melanogenesis appears to be continuous.

### SUMMARY OF THE INVENTION

It has now surprisingly been found, and this is the object of the present invention, that the spermidine compound, that is N-(3-aminopropyl)butan-1,4-diamine, as such or in the form of a pharmaceutically acceptable derivative such as a salt, is provided with a melanogenesis activity towards hair and can therefore be effectively used for promoting their pigmentation, in particular the shaft pigmentation.

Such activity allows configuring the use of the active compound in man as a natural pigmentation agent free from negative side effects, for example typical of hair dies.

The object of the invention is also a pharmaceutical or cosmetic or dietetic composition suitable to promote such pigmentation effect and therefore containing spermidine as active principle, as such or in the form of a pharmaceutically acceptable derivative such as a salt, for either topical or oral administration.

### DETAILED DESCRIPTION OF THE INVENTION

A preferred salt according to the invention is spermidine trichlorohydrate, namely N-(3-aminopropyl)butan-1,4-diamine ■ 3HCl.

A composition of the invention preferably comprises spermidine trichlorohydrate in a solution formulated for topical use. Suitable forms for topical use are, for example, a lotion, a conditioner, a shampoo, a mask.

A different composition of the invention preferably comprises spermidine trichlorohydrate in administration unit formulated for oral use. Suitable forms for oral use for example are a tablet or a capsule, either coated or not, or a granulate to disperse in water or other liquid.

Spermidine, as such or in the form of a pharmaceutically acceptable derivative, as a salt, is contained in a composition of the invention according to an amount preferably comprised within the following ranges:
- 10⁻⁷ to 1 g/100ml, corresponding to 0.004 to 4·10⁴ µM
- 10⁻⁵ to 1 g/100 ml, corresponding to 0.4 to 4·10⁴ µM
- 10⁻⁴ to 2.4·10⁻² g/100 ml, corresponding to 4 to 9·10² µM.

Further preferred concentration ranges are as follows:
- 10⁻⁶ to 10⁻¹ g/100 ml
- 10⁻⁵ to 10⁻² g/100 ml
- 10⁻⁴ to 10⁻³ g/100 ml
- 10⁻⁷ to 10⁻⁶ g/100 ml
- 10⁻⁶ to 10⁻⁵ g/100 ml
- 10⁻⁵ to 10⁻⁴ g/100 ml
- 10⁻⁴ to 10⁻³ g/100 ml
- 10⁻³ to 10⁻² g/100 ml
- 10⁻² to 10⁻¹ g/100 ml
- 10⁻¹ to 1 g/100 ml

The following formulation examples illustrate the invention, but are not intended to be limiting in any manner. The component amounts are expressed in grams or milligrams and in the case of examples 1 to 4, by concentration ranges.

### Example 1

### Shampoo

| Composition for 100 ml solution | | |
|---|---|---|
| Component (INCI nomenclature) | | |
| Magnesium laureth sulfate | 2 - 10 | g |
| Sodium lauroyl sarcosinate | 2 - 10 | g |
| Disodium laureth sulfosuccinate | 0.5 - 5 | g |
| PEG-200 hydrogenated glyceryl palmate | 0.5 - 5 | g |
| Cocamide MIPA | 0.5 - 5 | g |
| Glycol distearate | 0.5 - 5 | g |
| Glycerin | 0.5 - 5 | g |
| Laureth-7 | 0.1 - 3 | g |
| PEG-7 glyceryl cocoate | 0.1 - 3 | g |
| Lauryl methyl gluceth-10 hydroxypropyldimonium chloride | 0.1 - 3 | g |
| Polyquatemium-10 | 0.1 - 3 | g |
| Potassium undecilenoyl wheat protein | 0.1 - 3 | g |
| Panthenol | 0.1 - 3 | g |
| Tetrasodium EDTA | 0.1 - 3 | g |
| Spermidine trihydrochloride | 10⁻⁷ - 1 | g |
| Preservative | q.s. | |
| pH corrector (to a final pH of 5.0-5.5) | q.s. | |
| Parfum | q.s. | |
| Aqua | q.s. to 100 ml | |

### Example 2

### Hair mask

| Composition for 100 ml solution | | |
|---|---|---|
| Component (INCI) | | |
| Glycerin | 1 - 10 | g |
| Ammonium acrylolyl-dimethyltaurate / vp copolymer | 1 - 10 | g |
| Cyclopentasiloxane | 1 - 10 | g |
| Silicone quaternium-15 | 0.1 - 3 | g |
| Tocopheryl acetate | 0.1 - 3 | g |
| Dimethicone | 0.1 - 3 | g |
| Sericin | 0.1 - 3 | g |
| Methylparaben | 0.05 - 0.1 | g |
| C11-15 pareth-5 | 0.05 - 0.1 | g |
| C11-15 pareth-9 | 0.05 - 0.1 | g |
| Trideceth-12 | 0.05 - 0.1 | g |
| Decyl glucoside | 0.01 - 0.5 | g |
| Panthenyl ethyl ether | 0.01 - 0.5 | g |
| Disodium EDTA | 0.01 - 0.5 | g |
| Ethyl hexyl methoxycinnamate | 0.01 - 0.5 | g |
| Lactic acid | q.s. | |
| Preservative | q.s. | |
| Spermidine trihydrochloride | 10⁻⁷ - 1 | g |
| Parfum | q.s. | g |
| Aqua | q.s. to 100 ml | |

### Example 3

### Hair conditioner

| Composition for 100 ml solution | | |
|---|---|---|
| Component (INCI) | | |
| Cetearyl alcohol | 1 - 10 | g |
| Glyceryl stearate | 1 - 10 | g |
| Dimethicone | 1 - 10 | g |
| C12-13 alkyl lactate | 0.5 - 5 | g |
| Cetrimonium chloride | 0.5 - 5 | g |
| PEG-100 stearate | 0.5 - 5 | g |
| Cyclopentasiloxane | 0.5 - 5 | g |
| Hydroxyethylcellulose | 0.1 - 3 | g |
| Dimethiconol | 0.1 - 3 | g |
| Panthenol | 0.1 - 3 | g |
| Bis-isobutyl Peg/Ppg-20/35/amodimethicone copolymer | 0.05 - 2 | g |
| Phytantriol | 0.05 - 2 | g |
| Cetyl ethylhexanoate | 0.05 - 2 | g |
| Butylene glycol | 0.05 - 2 | g |
| Disodium edta | 0.05 - 2 | g |
| Polysorbate 80 | 0.05 - 2 | g |
| Sericin | 0.05 - 2 | g |
| Spermidine trihydrochloride | 10⁻⁷ - 1 | g |
| Preservative | q.s. | g |
| Parfum | q.s. | g |
| Aqua | q.s. to 100 ml | |

### Example 4

### Hair lotion

| Composition for 100 ml solution | | |
|---|---|---|
| Component (INCI) | | |
| Alcohol | 10 - 20 | g |
| PEG-40 Hydrogenated Castor Oil | 0.2 - 2 | g |
| Disodium EDTA | 0.01 - 0.5 | g |
| Parfum | q.s. | |
| Spermidine trihydrochloride | 10⁻⁷ - 1 | g |
| Aqua | q.s. to 100 ml | |

### Example 5

### Hard gelatine capsules

| Composition for a single capsule | | |
|---|---|---|
| Component | | |
| Lactose Monohydrate | 85 | mg |
| Corn starch | 25 | mg |
| Talc | 5 | mg |
| Spermidine trihydrochloride | 1.25 | mg |
| Magnesium stearate | 1.5 | mg |
| Hard gelatine capsule shell N. 4 | 1 | n. |

### Example 6

### Soft gelatine capsules

| Composition for a single capsule | | |
|---|---|---|
| Component | | |
| Soy oil | 263.07 | mg |
| Gelatine | 129.7 | mg |
| Glycerine | 36.5 | mg |
| Sorbitol 70% | 24.2 | mg |
| Water | 23.274 | mg |
| Yellow wax | 22 | mg |
| Fatty acids mono-diglycerides | 20 | mg |
| Soy lecithin | 10 | mg |
| Titanium Dioxide | 0.686 | mg |
| Spermidine trihydrochloride | 0.25 | mg |

### Example 7

### Tablet

| Composition for a single tablet | | |
|---|---|---|
| Component | | |
| Microcrystalline cellulose | 168.97 | mg |
| Lactose | 150 | mg |
| Methylcellulose | 45 | mg |
| Mono- and diglycerides of fatty acids | 9 | mg |
| Colloidal silicon dioxide | 8 | mg |
| Magnesium stearate | 2 | mg |
| Spermidine trihydrochloride | 1.0 | mg |

### Example 8

### Slow-release coated tablet

| Composition for a single tablet | | |
|---|---|---|
| Component | | |
| Microcrystalline cellulose | 105 | mg |
| Calcium Phosphate bibasic dihydrate | 85 | mg |
| Hydroxypropylmethylcellulose K100 | 45 | mg |
| Sepifilm TM LP 770 white | 15 | mg |
| Magnesium stearate | 8 | mg |
| Hydroxypropylmethylcellulose | 6 | mg |
| Colloidal silicon dioxide | 3.5 | mg |
| Spermidine trihydrochloride | 0.55 | mg |

### Example 9

### Effervescent granulate in sachet for making an improptu solution

| Composition for a single sachet | | |
|---|---|---|
| Component | | |
| Mannitol | 960 | mg |
| Tartaric acid | 530 | mg |
| Anhydrous sodium bicarbonate | 280 | mg |
| Flavor | 130 | mg |
| Polyvinyl pyrrolidone | 45 | mg |
| Trometamol | 32 | mg |
| Aspartame | 20 | mg |
| Spermidine trihydrochloride | 1.25 | mg |
| Anhydrous colloidal silicon | 2 | mg |

Below is the description of an experimental study relating to the activity in the use of spermidine according to the present invention.

### ACTIVITY STUDY

### Tissue samples

The skin of normal human scalp was taken from a woman who had undergone a routine face lifting surgery after receiving the informed consent. All the experiments were carried out according to the Helsinki principles, with the ethical committee's approval.

### Skin organ culture with complete thickness

The tissues subject to biopsy with 3-4 mm cylindrical scalpel were cultured at 37°C for 6 days in Williams E medium (Biochrom, Cambridge, U.K.), integrated with 100 IU ml⁻¹ penicillin, 10 µg ml⁻¹ streptomycin (Gibco, Karlsruhe, Germany), 10 µg ml⁻¹ insulin (Sigma, Taukfirchen, Germany), 10 ng ml⁻¹ hydrocortisone (Sigma) and 2mmol L⁻¹ (L-glutamine (Invitrogen, Paisley, U.K.).

Spermidine trichlorohydrate, or the vehicle as a reference substance, was then administered at a concentration of 0.1 µM, once at each medium change (i.e., every 48 hours).

### Micro-dissection of hair follicles and organ culture

The hair follicles (HF) in anagen VI phase with normal pigmentation (gray/white hair follicles were excluded from the study) were micro-dissected from normal human scalp skin and subject to organ culture based on the Philpott model. Spermidine or the vehicle were administered once at each medium change (i.e., every 48 hours).

### LDH measurement

The LDH activity in the supernatant served as a cytotoxicity indicator and was measured every day according to the manufacturer's instructions (Cytotoxicity Detection Kit; Roche, Mannheim, Germany). The sample absorbance was measured at 490 nm using an ELISA plate reader.

### Hair shaft elongation

The measurements of the hair follicle shaft length were taken every second day on the single hair follicles using a Zeiss inverted binocular microscope with an ocular measurement reticle.

### Determination of the hair follicle cycle stage

The determination of the hair follicle cycle stage was carried out based on the morphological criteria defined before, and the percentage of hair follicles in anagen phase and in early, intermediate or late catagen phase was determined.

### Hair pigmentation

The Masson-Fontana staining was carried out for the histochemical display of melanin on frozen sections. Melanin was stained in the form of brown granules and the pigmentation level was determined through the quantitative Masson-Fontana technique (Ito N., Ito T., Kromminga A., Bettermann A., Takigawa M., Kees F., Straub R. H., and Paus R. (2005): Human hair follicles display a functional equivalent of the hypothalamic-pituitary-adrenal axis and synthesize cortisol. FASEB J 19, 1332-4).

This method is a particularly sensitive and reliable indicator of melanin synthesis variations, as proven by enzymatic activity assays and standard tyrosinase expression (Kauser S., Slominski A., Wei E. T., and Tobin D. J. (2006): Modulation of the human hair follicle pigmentary unit by corticotropin-releasing hormone and urocortin peptides. FASEB J 20, 882-95).

The staining intensity was analyzed in a defined reference region of the hair follicle pigmentation unit using the ImageJ software (National Institute of Health).

### Proliferation and apoptosis measurement

In order to evaluate the apoptotic cells in co-location with a proliferation marker Ki-67, the TUNEL (terminal dUTP nick-end labeling) dual staining method with Ki-67 was used. The cryostat sections were fixed in paraformaldehyde and ethanolacetic acid (2:1) and marked with a digoxigenin-deoxy-UTP (kit for the identification of apoptosis in situ with ApopTag fluorescein; Intergen, Purchase, NY) in the presence of terminal deoxynucleotidyl transferase, followed by incubation with a murine anti-Ki-67 antiserum (1:20 in PBS overnight at 4°C; Dako, Glostrup, Denmark). TUNEL-positive cells were displayed by a conjugate isothiocyanate fluorescein anti digoxigenin antibody (kit ApopTag), whereas Ki-67 was detected by a goat anti-mouse antibody marked with rhodamine (Jackson ImmunoResearch, West Grove, PA). Negative controls were carried out omitting the terminal deoxynucleotidyl transferase and the Ki-67 antibody. The counter-staining was carried out with 4',6-diamidino-2-phenylindole (DAPI) (Roche Molecular Biochemicals GmbH, Mannheim, Germany). The quantitative histomorphometric assessment was carried out; Ki-67, TUNEL or DAPI-positive cells were counted in a reference region defined beforehand of the hair follicle and skin matrix and the percentage of positive Ki-67/TUNEL cells was determined.

### Quantitative immunohistochemistry of K15

The tyramide signal amplification method described before was used for examining the expression of keratin K15 (Kloepper et al., 2008). In brief, cryosections fixed with acetone were washed three times for 5 minutes using the TNT (tris-HCL NaCl Tween) buffer (0.1 mol/l Tris-HCl, pH 7.5; containing 0.15 mol/l NaCl and 0.05% Tween 20). Radish peroxidase was then blocked through wash with 3% H₂O₂ in an isotonic phosphate buffer (PBS) for 15 minutes. Preincubation was carried out with the incubation of avidin and biotin for 15 minutes and with 5% normal goat serum in TNT for 30 minutes with intermediate washing steps. Murine anti-human K15 (clone LHK15, Chemicon, Billerica, USA) were diluted in TNT and incubated overnight at 4°C, followed by a secondary goat anti-mouse biotinylate antibody (1:200 in TNT) for 45 minutes at room temperature. Radish streptavidin-peroxidase was then administered (kit TSA; Perkin-Elmer, Boston, MA, USA) (1:100 in TNT) for 30 minutes at room temperature. The reaction was amplified with a FITC-tyramide amplification agent at room temperature for 5 minutes (1:50 in an amplification diluent supplied with the kit). The intensity of this immuno-staining was quantified by the ImageJ (National Institutes of Healt) software. The staining intensity of reference regions defined in hair follicles was measured and compared between the control groups treated with vehicle only and the groups treated with spermidine.

### Statistical analysis

The statistical analysis was carried out using a bilateral Student t-test for unpaired samples.

### Results

The figures of the annexed drawings show the results of the experimental study described.

### BRIEF DESCRIPTION OF THE FIGURES

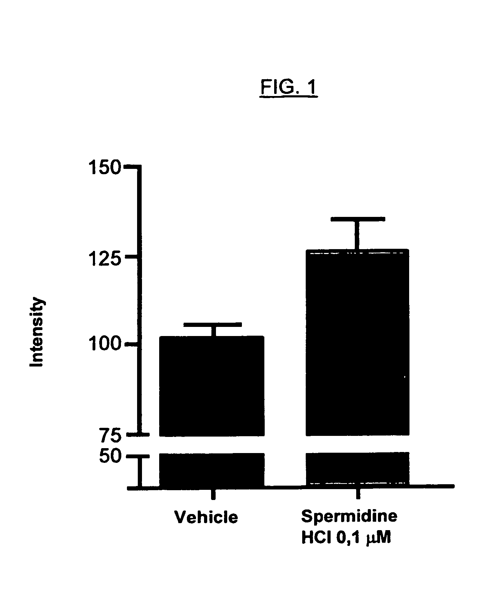

Fig. 1 shows a diagram relating to the pigmentation intensity in hair follicles as measured and compared between the control group treated with vehicle only and the group treated with spermidine 3HCl in a concentration of 0.1 µM.

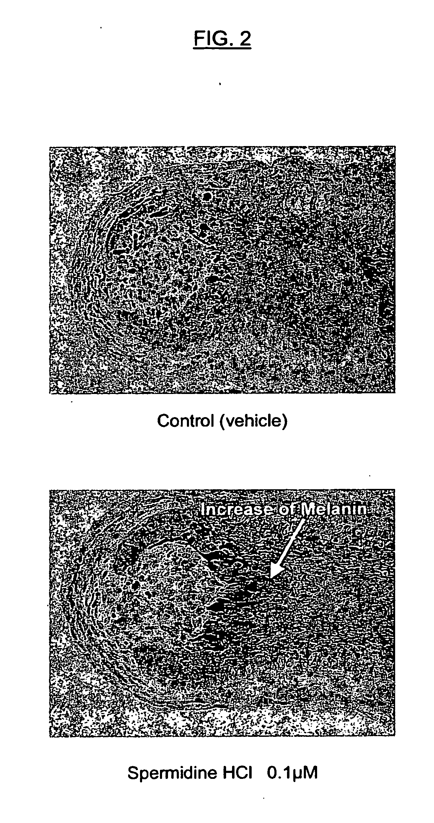

Fig. 2 shows the corresponding images taken from the hystochemical display of melanin through Masson-Fontana staining.

The increase of melanin is clear from both figures in the case of treatment with spermidine, therefore a significant melanogenesis activity towards hair treated with such compound compared to the reference vehicle.

## Claims

1. Use of spermidine, as such or in the form of a pharmaceutically acceptable derivative, as an active principle to promote hair pigmentation, in particular the pigmentation of the hair shaft.

2. Use according to claim 1, wherein spermidine is in the form of spermidine trichlorohydrate, namely N-(3-aminopropyl)butan-1,4-diamine - 3HCl.

3. Use according to claim 1, wherein spermidine is formulated in a composition with any suitable excipient for topical administration on the scalp.

4. Use according to claim 3, wherein spermidine is formulated as a lotion or a balsam or a shampoo or a hair mask.

5. Use according to claim 1, wherein spermidine is formulated in a composition with any suitable excipient for oral administration.

6. Use according to claim 5, wherein spermidine is formulated as a coated or uncoated tablet, or a hard or soft capsule, or a granulate.

7. Use according to claims 1, 2 and 3, wherein spermidine is formulated in a composition in an amount in the range between 0.004 to 4-10⁴ µM, corresponding to an amount of spermidine trichlorohydrate in the range from 10⁻⁷ to 1g/100 ml.

8. Use according to claim 7, wherein spermidine is formulated in an amount in the range between 0.4 to 4-10⁴ µM, corresponding to an amount of spermidine trichlorohydrate in the range from 10⁻⁵ to 1g/100 ml.

9. Use according to claim 8, wherein spermidine is formulated in an amount in the range between 3.9 to 9.4-10² µM, corresponding to an amount of spermidine trichlorohydrate in the range from 10⁻⁴ to 2.4-10⁻² g/100 ml.

10. Use according to claim 5, wherein spermidine is formulated in a composition for oral administration in an amount in the range between 0.14 and 0.71 mg per administration unit.

11. Use according to claims 2 and 5, wherein spermidine trichlorohydrate is formulated in a composition for oral administration in an amount in the range between 0.25 to 1.25 mg per administration unit.

## Patentansprüche

1. Verwendung von Spermidin als solches oder in Form eines pharmazeutisch unbedenklichen Derivats als Wirkstoff zur Förderung der Haarpigmentierung, insbesondere der Pigmentierung des Haarschafts.

2. Verwendung nach Anspruch 1, wobei Spermidin in Form von Spermidin-trihydrochlorid, nämlich N-(3-Aminopropyl)butan-1,4-diamin•3HCl, vorliegt.

3. Verwendung nach Anspruch 1, wobei Spermidin in einer Zusammensetzung mit einem geeigneten Hilfsstoff für die topische Verabreichung auf die Kopfhaut formuliert ist.

4. Verwendung nach Anspruch 3, wobei Spermidin als Lotion oder Balsam oder Shampoo oder Haarmaske formuliert ist.

5. Verwendung nach Anspruch 1, wobei Spermidin in einer Zusammensetzung mit einem geeigneten Hilfsstoff für die orale Verabreichung formuliert ist.

6. Verwendung nach Anspruch 5, wobei Spermidin als beschichtete oder unbeschichtete Tablette oder Hart- oder Weichkapsel oder Granulat formuliert ist.

7. Verwendung nach den Ansprüchen 1, 2 und 3, wobei Spermidin in einer Zusammensetzung in einer Menge im Bereich zwischen 0,004 bis 4·10⁴ µM, was einer Menge von Spermidin-trihydrochlorid von 10⁻⁷ bis 1 g/100 ml entspricht, formuliert ist.

8. Verwendung nach Anspruch 7, wobei Spermidin in einer Zusammensetzung in einer Menge im Bereich zwischen 0,4 bis 4·10⁴ µM, was einer Menge von Spermidin-trihydrochlorid von 10⁻⁵ bis 1 g/100 ml entspricht, formuliert ist.

9. Verwendung nach Anspruch 8, wobei Spermidin in einer Zusammensetzung in einer Menge im Bereich zwischen 3,9 bis 9,4·10² µM, was einer Menge von Spermidin-trihydrochlorid von 10⁻⁴ bis 2,4·10⁻² g/100 ml entspricht, formuliert ist.

10. Verwendung nach Anspruch 5, wobei Spermidin in einer Zusammensetzung für die orale Verabreichung in einer Menge im Bereich zwischen 0,14 und 0,71 mg pro Verabreichungseinheit formuliert ist.

11. Verwendung nach den Ansprüchen 2 und 5, wobei Spermidin-trihydrochlorid in einer Zusammensetzung für die orale Verabreichung in einer Menge im Bereich zwischen 0,25 und 1,25 mg pro Verabreichungseinheit formuliert ist.

## Revendications

1. Utilisation de spermidine, en tant que ou sous la forme d'un dérivé acceptable du point de vue pharmaceutique, comme principe actif pour encourager la pigmentation capillaire, en particulier la pigmentation de la tige du cheveu.

2. Utilisation selon la revendication 1, dans laquelle la spermidine est sous la forme de trichloro-hydrate de spermidine, à savoir de N-(3-aminopropyle)butane-1,4-diamine·3HCl.

3. Utilisation selon la revendication 1, dans laquelle la spermidine est formulée dans une composition avec n'importe quel excipient approprié pour l'administration topique sur le cuir chevelu.

4. Utilisation selon la revendication 3, dans laquelle la spermidine est formulée comme une lotion ou comme un baume ou un shampooing ou un masque pour cheveux.

5. Utilisation selon la revendication 1, dans laquelle la spermidine est formulée dans une composition avec n'importe quel excipient approprié pour l'administration orale.

6. Utilisation selon la revendication 5, dans laquelle la spermidine est formulée comme un comprimé enrobé ou non enrobé, ou une capsule dure ou molle ou un granulé.

7. Utilisation selon les revendications 1, 2 et 3, dans laquelle la spermidine est formulée dans une composition en une quantité dans la plage entre 0,004 à 4·10⁴ µM, correspondant à une quantité de spermidine trichlorohydrate dans la plage de 10⁻⁷ à 1g/100 ml.

8. Utilisation selon la revendication 7, dans laquelle la spermidine est formulée en une quantité dans la plage entre 0,4 à 4·10⁴ µM, correspondant à une quantité de spermidine trichlorohydrate dans la plage de 10⁻⁵ à 1g/100 ml.

9. Utilisation selon la revendication 8, dans laquelle la spermidine est formulée en une quantité dans la plage entre 3,9 à 9,4·10² µM, correspondant à une quantité de spermidine trichlorohydrate dans la plage de 10⁻⁴ à 2,4·10⁻² g/100 ml.

10. Utilisation selon la revendication 5, dans laquelle la spermidine est formulée dans une composition pour l'administration orale en une quantité dans la plage entre 0,14 et 0,71 mg par unité d'administration.

11. Utilisation selon les revendications 2 et 5, dans laquelle le spermidine trichlorohydrate est formulé dans une composition pour l'administration orale en une quantité dans la plage entre 0,25 à 1,25 mg par unité d'administration.
